# EUROPÄISCHE PATENTSCHRIFT

(11) **EP 2 435 441 B1**
(45) Veröffentlichungstag und Bekanntmachung des Hinweises auf die Patenterteilung: **06.01.2016**
(21) Anmeldenummer: 10723984.0
(22) Anmeldetag: 25.05.2010
(51) Int. Cl.: C07D 501/04, C07D 501/18, C07D 501/56

(54) **VERFAHREN ZUR HERSTELLUNG VON CEFTOBIPROL MEDOCARIL**
METHOD FOR PRODUCING CEFTOBIPROL MEDOCARIL
PROCÉDÉ DE FABRICATION DE CEFTOBIPROL MEDOCARIL

(30) Priorität: 25.05.2009 EP 09161028
(43) Veröffentlichungstag der Anmeldung: 04.04.2012
(73) Patentinhaber: Sandoz AG, 4056 Basel (CH)
(72) Erfinder: KREMMINGER, Peter, A-6250 Kundl (AT); LUDESCHER, Johannes, A-6250 Kundl (AT); STURM, Hubert, A-6250 Kundl (AT)
(74) Vertreter: Herzog, Fiesser & Partner Patentanwälte PartG mbB
(86) Internationale Anmeldenummer: PCT/EP2010/057104
(87) Internationale Veröffentlichungsnummer: WO 2010/136422

(56) Entgegenhaltungen:
- EP-A- 0 849 269
- EP-B- 1 087 980
- WO-A-95/29182
- WO-A1-01/90111

## Beschreibung

Die vorliegende Erfindung betrifft ein Verfahren zur Herstellung von organischen Verbindungen, insbesondere von Natrium (6R,7R)-7-[(Z)-2-(5-Amino-[1,2,4]thiadiazol-3-yl)-2-hydroxyimino-acetylamino]-8-oxo-3-[(E)-(R)-1'-(5-methyl-2-oxo-[1,3]-dioxol-4-ylmethoxycarbonyl)-2-oxo-[1,3']bipyrrolidinyl-3-ylidenemethyl]-5-thia-1-aza-bicyclo[4.2.0]oct-2-ene-2 carboxylat (Ceftobiprol Medocaril), bzw. Verbindungen der allgemeinen Formel (1) und der allgemeinen Formel (2), die Verbindungen selbst sowie Intermediate in der erfindungsgemäßen Herstellung. Ceftobiprol Medocaril ist ein parenterales Cephalosporin mit hervorragenden antibakteriellen Eigenschaften. Einen Überblick gibt beispielsweise Current Opinion in Pharmacology 2006, 6, 480-485.

Verfahren zur Herstellung von Ceftobiprol Medocaril sind an sich bekannt. Den aus dem Stand der Technik bekannten Verfahren ist gemeinsam, dass ausgehend von 7-Aminocephalosporansäure eine Vielzahl an Zwischenstufen hergestellt, isoliert und aufgereinigt werden müssen, um Ceftobiprol Medocaril der allgemeinen Formel (1) in ausreichender Reinheit zu erhalten.

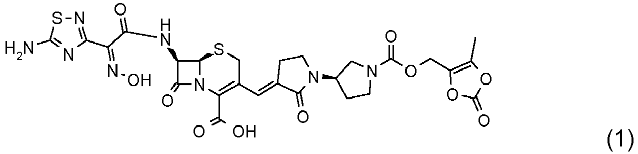

Die Verbindung der allgemeinen Formel (1) ist an sich bekannt und beispielsweise in WO 99/65920 beschrieben. Sie ist verwendbar für die Behandlung und zur Prophylaxe von bakteriellen Infektionskrankheiten, insbesonders von Infektionskrankheiten, die durch Methicillin resistente Staphylococcus Aureus Stämme hervorgerufen werden.

WO 99/65920 beschreibt als letzten Schritt des Herstellprozesses von Ceftobiprol Medocaril eine Umsetzung, wobei bei einer Verbindung der allgemeinen Formel (2) die Medocaril-Prodrug Einheit eingeführt wird.

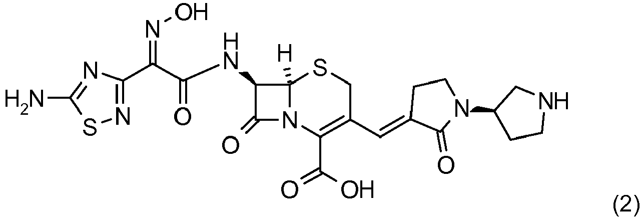

Die Verbindung der allgemeinen Formel (2) ist ebenfalls an sich bekannt und wurde beispielsweise in EP 0 849 269 A1 beschrieben. Die Herstellung der Verbindung der allgemeinen Formel (2) erfolgt gemäß EP 0 849 269 A1 ausgehend von (2R,6R,7R)-tert. Butoxycabonylamin o-3-formyl-8-oxo-5-thia-1-azabicyclo[4.2.0]oct-3-ene-2-carboxylic acid benzhydrylester durch Wittig-Reaktion mit (1'-Allyloxycarbonyl-2-oxo-[1,3']bipyrrolidinyl-3-yl)-triphenylphosphonium bromid. Das dabei entstehende Δ2-Reaktionsprodukt wird durch Sulfoxidierung und anschließender Reduktion zum gewünschten Δ3-Isomeren rückisomerisiert und danach mit Trifluoressigsäure vom Benzhydrylester entschützt. Die Acylierung in Position 7 erfolgt durch Reaktion mit (Z)-(5-Amino-[1,2,4]-thiadiazol-3-yl)-trityloxyiminothioessigsäure S-benzothiazol-2-yl ester. Die Verbindung der allgemeinen Formel (2) wird im Anschluss durch Abspaltung der Schutzgruppen erhalten.

In EP 1 067 131 A1 wird die Bildung des Ylids in Toluol oder einer Mischung aus Toluol und Dichlormethan durch Zugabe von Alkali tert. Butylat in Tetrahydrofuran beschrieben, wodurch die Base als Lösung zugegeben werden kann. Die Umsetzung des Ylids mit dem entsprechenden Aldehyd ist bei einer Reaktionstemperatur von - 70°C beschrieben.

EP 0 841 339 A1 betrifft Cephalosporin Derivate sowie Verfahren zu deren Herstellung. WO 95/29182 offenbart ebenfalls Zwischenprodukte für die Herstellung von Cephalosporinen.

WO 01/90111 beschreibt eine weitere Herstellung von Ceftobiprol Medocaril in mehreren Stufen ausgehend von Desacetyl-7-aminocephalosporansäure durch Acylierung mit (Z)-(5-Amino-[1,2,4]-thiadiazol-3-yl)-trityloxyiminothioessigsäure S-benzothiazol-2-yl ester in N,N-Dimethylformamid, gefolgt von in situ Veresterung mit Diphenyldiazomethan in Dichlormethan zum entsprechenden Benzhydrylester, der durch Zugabe von Hexan gefällt und isoliert wird. Dieses Produkt wird im nächsten Schritt mit TEMPO/ NaOCl in Dichlormethan/Wasser oder mit Braunstein in Tetrahydrofuran/Dichlormethan zum entsprechenden Aldehyd oxidiert. Der nächste Reaktionschritt beinhaltet die Wittig-Reaktion zum 3-Vinylsubstituierten Derivat, bei der in Dichlormethan/Toluol/Tetrahydrofuran bei -78°C umgesetzt wird. Das Rohprodukt wird mit Ethanol ausgerührt und aus Dichlormethan/tert. Butylmethylether umkristallisiert bzw. chromatographisch gereinigt. Gemäß dem in WO 01/90111 offenbarten Verfahren wird die Wittigreaktion bei tiefen Temperaturen von -80 bis - 70°C in einem komplexen Lösungsmittelgemisch aus Dichlormethan, Toluol und Tetrahydrofuran durchgeführt. Dies führt bei der Durchführung der Reaktion im Produktionsmaßstab zu erheblichen Nachteilen, da eine Regenerierung der Prozesslösungsmittel erschwert ist.

Nachteilig an den aus dem Stand der Technik bekannten Synthesen ist, dass die Verbindung der allgemeinen Formel (2) bzw. der allgemeinen Formel (1) über einen vielstufigen Prozeß hergestellt wird, der komplexe Syntheseschritte beinhaltet und schlechte Gesamtausbeuten liefert. Darüber hinaus sind aufwendige Schutzgruppenoperationen nötig.

Es wurde nun überraschenderweise gefunden, dass Verbindungen der allgemeinen Formel (1) bzw. der allgemeinen Formel (2) über ein Verfahren hergestellt werden können, das mit wenigen Stufen auch im industriellen Maßstab durchführbar ist.

Soweit nicht explizit ausgeführt betreffen die folgenden Ausführungen im Rahmen der vorliegenden Erfindung jeweils die genannten Verbindungen selbst sowie deren pharmazeutisch verträgliche Salze.

Die vorliegende Erfindung betrifft demgemäß ein Verfahren zur Herstellung einer Verbindung der allgemeinen Formel (1) 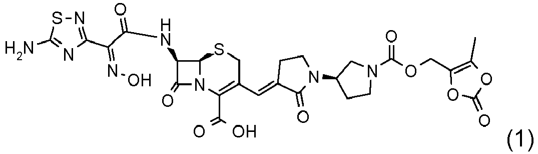 mindestens umfassend die folgenden Schritte (a), (b) und (c):
(a) Umsetzung einer Verbindung der allgemeinen Formel (3) 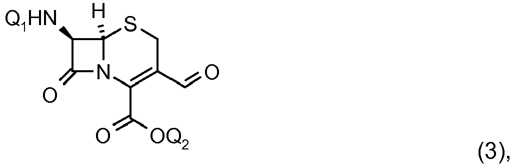 wobei Q1 und Q2 unabhängig voneinander für eine Silylgruppe steht oder wobei Q1 für ein Wasserstoffatom und Q2 für eine Silylgruppe steht,
   mit einer Verbindung der allgemeinen Formel (4) 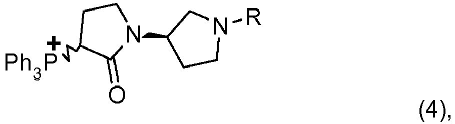 wobei R für eine 5-Methyl-2-oxo-[1,3]-dioxol-4-ylmethylcarbonyl-gruppe steht,
   zu einer Verbindung der allgemeinen Formel (5) 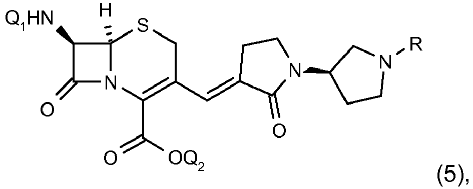 wobei Q₁, Q₂ und R wie oben definiert sind;
(b) Umsetzung der Verbindung der allgemeinen Formel (5) mit einer Verbindung der allgemeinen Formel (6) 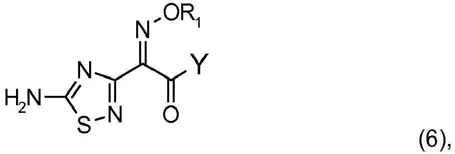 wobei R₁ für eine Hydroxy-Schutzgruppe und Y für eine aktivierende Funktionalität steht,
   gegebenenfalls nach Entfernung der Schutzgruppe, falls Q₁ für eine Silylgruppe steht,
   zu einer Verbindung der allgemeinen Formel (7) 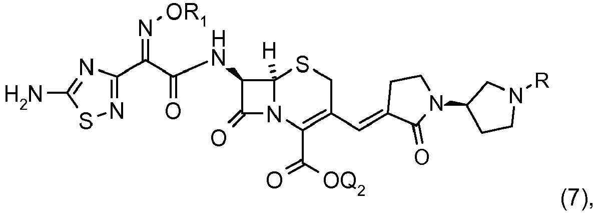 wobei R₁, Q₂ und R wie oben definiert sind; und
(c) Umsetzung der Verbindung der allgemeinen Formel (7) zu der Verbindung der allgemeinen Formel (1).

Überraschenderweise wurde gefunden, dass die Verbindung der Formel (4) wobei R für eine 5-Methyl-2-oxo-[1,3]-dioxol-4-ylmethylcarbonyl-gruppe steht direkt in den Schritt (a) eingesetzt werden kann. Man hätte erwarten können, dass die Esterbindung des medocaril Restes bei den basischen Bedingungen dieses Schrittes größere Labilität zeigen würde.

Das erfindungsgemäße Verfahren umfasst mindestens die Schritte (a), (b) und (c). Das erfindungsgemäße Verfahren kann darüber hinaus auch weitere Schritte umfassen, beispielsweise Schutzgruppenoperationen. Dabei ist es erfindungsgemäß möglich, dass diese weiteren Schritten vor oder nach den Schritten (a), (b) und (c) erfolgen. Ebenso ist es jedoch möglich, dass die weiteren Schritte zwischen den Schritten (a) und (b) oder zwischen den Schritten (b) und (c) erfolgen. Das erfindungsgemäße Verfahren ermöglicht die Herstellung der Verbindung der allgemeine Formel (1) in einfacher Weise und in hoher Reinheit.

Das erfindungsgemäße Verfahren umfasst Schritt (a), d.h. die Umsetzung einer Verbindung der allgemeinen Formel (3) 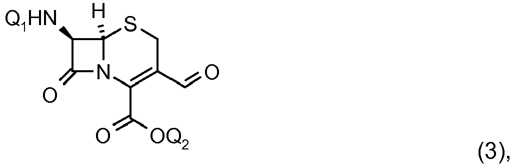 wobei Q1 und Q2 unabhängig voneinander für eine Silylgruppe steht oder wobei Q1 für ein Wasserstoffatom und Q2 für eine Silylgruppe steht,
mit einer Verbindung der allgemeinen Formel (4) 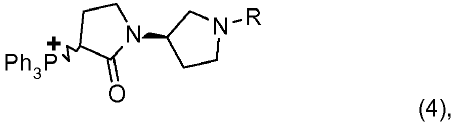 wobei R für eine 5-Methyl-2-oxo-[1,3]-dioxol-4-ylmethylcarbonyl-gruppe (DMDO) steht,
zu einer Verbindung der allgemeinen Formel (5) 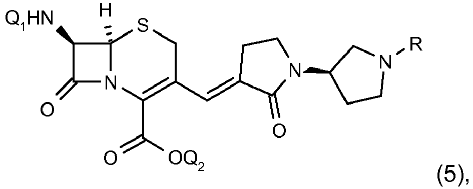 wobei Q₁, Q₂ und R wie oben definiert sind.

Erfindungsgemäß kann die Umsetzung gemäß Schritt (a) in jeder dem Fachmann bekannten Weise durchgeführt werden. Dabei wird die Verbindung der allgemeinen Formel (3) mit dem Phosphoniumsalz der allgemeinen Formel (4) umgesetzt. Die Umsetzung erfolgt erfindungsgemäß in Gegenwart einer Base unter Bildung der Verbindung der allgemeinen Formel (5).

Geeignete Reaktionsbedingungen und Lösungsmittelsysteme sind beispielsweise in WO 95/29182 auf Seite 26, zweiter Absatz bis Seite 27, einschließlich vorletzter Absatz beschrieben, sowie in Beispiel 31 auf Seite 42 in WO 95/29182.

Die Umsetzung gemäß Schritt (a) kann erfindungsgemäß beispielsweise in Gegenwart eines Silylierungsmittels und/oder eines Epoxids durchgeführt werden, insbesondere in Gegenwart von BSA und/oder Propylenoxid.

Daher betrifft die vorliegende Erfindung auch ein Verfahren zur Herstellung einer Verbindung der allgemeinen Formel (1) wie oben beschrieben, wobei Schritt (a) in Gegenwart eines Silylierungsmittels und eines Epoxids durchgeführt wird.

Gemäß bevorzugter Ausführungsformen betrifft die vorliegende Erfindung auch ein Verfahren zur Herstellung einer Verbindung der allgemeinen Formel (1) wie oben beschrieben, wobei es sich bei dem Silylierungsmittel um BSA handelt oder wobei es sich bei dem Epoxid um Propylenoxid handelt.

Die erfindungsgemäßen Verfahren umfassen darüber hinaus einen Schritt (b), d.h. die Umsetzung der Verbindung der allgemeinen Formel (5) mit einer Verbindung der allgemeinen Formel (6) 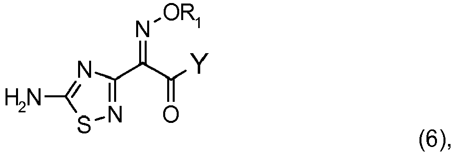 wobei R₁ für eine Hydroxy-Schutzgruppe und Y für eine aktivierende Funktionalität steht,
gegebenenfalls nach Entfernung der Schutzgruppe, falls Q₁ für eine Silylgruppe steht,
zu einer Verbindung der allgemeinen Formel (7) 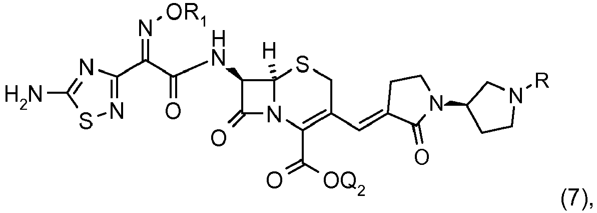 wobei R₁, Q₂ und R wie oben definiert sind.

Gemäß Schritt (b) des erfindungsgemäßen Verfahrens wird die Verbindung der allgemeinen Formel (5) mit der Verbindung der allgemeinen Formel (6) acyliert.

Erfindungsgemäß ist Y eine aktivierende Funktionalität wie beispielsweise ein Halogenid, wobei geeignete Halogenide beispielsweise in J. Antibiotics 37:557 - 571, 1984 offenbart werden, ein gemischtes Anhydrid, wobei geeignete gemischte Anhydride beispielsweise in Yakugaku Zasshi 110 (9) 658-664, 1990 offenbart werden, oder eine Gruppe ausgewählt aus den Gruppen (s), (t) und (u):

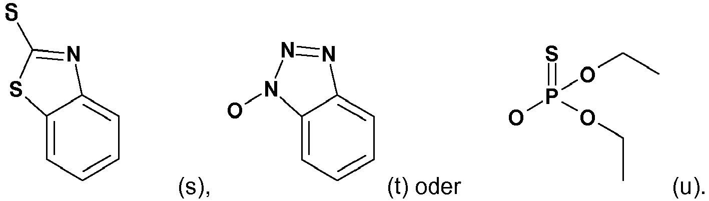

Dabei kann die Umsetzung gemäß Schritt (b) des erfindungsgemäßen Verfahrens grundsätzlich auf jede dem Fachmann bekannte geeignete Art durchgeführt werden.

Geeignete Reaktionsbedingungen und Lösungsmittelsysteme sind beispielsweise in EP 37380 A2 von Seite 8, Zeile 16 bis Seite 9, Zeile 5 beschrieben.

Erfindungsgemäß ist es bevorzugt, dass für die Umsetzung gemäß Schritt (b) die Verbindung der allgemeinen Formel (5) durch Silylierung oder Salzbildung gelöst wird, und nach erfolgter Acylierung mit der Verbindung der allgemeinen Formel (6) die Schutzgruppen in einem Schritt abgespalten werden.

Dabei kann im Rahmen der vorliegenden Erfindung Schritt (b) ohne Zwischenisolierung bzw. im Eintopfverfahren durchgeführt werden.

Gemäß einer bevorzugten Ausführungsform betrifft die vorliegende Erfindung daher auch ein Verfahren zur Herstellung einer Verbindung der allgemeinen Formel (1) wie oben beschrieben, wobei für die Umsetzung gemäß Schritt (b) die Verbindung der allgemeinen Formel (5) durch Silylierung oder Salzbildung gelöst wird, und nach erfolgter Acylierung mit der Verbindung der allgemeinen Formel (6) die Schutzgruppen in einem Schritt abgespalten werden.

Gemäß einer weiteren Ausführungsform betrifft die vorliegende Erfindung auch ein Verfahren zur Herstellung einer Verbindung der allgemeinen Formel (1) wie oben beschrieben, wobei Schritt (b) ohne Zwischenisolierung bzw. im Eintopfverfahren durchgeführt wird.

Gemäß Schritt (c) erfolgt die Umsetzung der allgemeinen Formel (7) zu der Verbindung der allgemeinen Formel (1), nach Entfernung der Schutzgruppe Q₂.

Es wurde überraschenderweise gefunden, dass mittels dieses erfindungsgemäßen Verfahrens eine Reduktion der Anzahl der isolierten Zwischenstufen bei der Herstellung von Verbindung der allgemeinen Formel (1), insbesondere von Ceftobiprol Medocaril, erhalten werden kann, was letztlich zu höheren Gesamtausbeuten und in Folge zu günstigeren Herstellkosten führt.

Die gemäß dem erfindungsgemäßen Verfahren erhaltenen Verbindungen zeichnen sich unter anderem durch eine hohe Reinheit aus.

Die vorliegende Erfindung betrifft auch neue Intermediate der Synthese. So betrifft die vorliegende Erfindung gemäß einem weiteren Aspekt eine Verbindung der allgemeinen Formel (5) 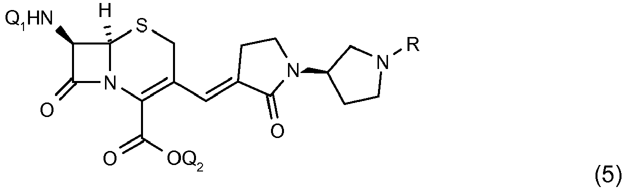 wobei
R für eine 5-Methyl-2-oxo-[1,3]-dioxol-4-ylmethylcarbonyl-gruppe steht und
wobei Q1 und Q2 unabhängig voneinander für eine Silylgruppe steht oder wobei Q1 für ein Wasserstoffatom und Q2 für eine Silylgruppe steht, sowie Salze davon, insbesondere organische Aminsalze, wie z.B. das Dicyclohexylaminsalz aus Beispiel 3, oder insbesondere Salze mit starken organischen Säuren, wie das Trifluoressigsäuresalz oder das Tosylat oder Mesylat.

Erfindungsgemäß kann R für eine 5-Methyl-2-oxo-[1,3]-dioxol-4-ylmethylcarbonyl-gruppe stehen. Demgemäß betrifft die vorliegende Erfindung gemäß einer weiteren Ausführungsform eine Verbindung der allgemeinen Formel (5) wie oben beschrieben, wobei Q₁ für eine Trimethylsilylgruppe oder ein Wasserstoffatom und Q₂ für eine Trimethylsilylgruppe und R für eine 5-Methyl-2-oxo-[1,3]-dioxol-4-ylmethylcarbonylgruppe steht.

Die Verbindung der allgemeinen Formel (5) ist eine wesentliche Zwischenstufe des erfindungsgemäßen Verfahrens und ermöglicht die einfache Synthesesequenz des erfindungsgemäßen Verfahrens.

Die vorliegende Erfindung betrifft daher auch die Verwendung einer Verbindung der allgemeinen Formel (5) wie oben beschrieben zur Herstellung einer Verbindung der allgemeinen Formel (1). Darüber hinaus betrifft die vorliegende Erfindung auch die Verwendung einer Verbindung der allgemeinen Formel (5) wie oben beschrieben zur Herstellung einer Verbindung der allgemeinen Formel (1).

Im Folgenden wird die vorliegende Erfindung anhand von Beispielen näher erläutert.

### 1. Referenzbeispiel:

### (6R,7R)-7-Amino-3[E-(R)-1'-(5-methyl-2-oxo-[1,3]-dioxol-4-ylmethoxycarbonyl)-2-oxo-[1,3']bipyrrolidinyl-3-ylidenemethyl]-8-oxo-5-thia-1-aza-bicyclo[4.2.0]oct-2-ene-2-carbonsäure

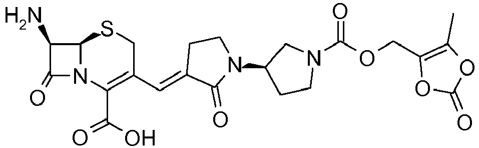

1.95g 7-Amino-3-formyl-ceph-3-em-4-carboxylat wurden in 13.0ml Bis(trimethylsilyl)acetamid und 23ml Propylenoxid gelöst. Anschließend wurden bei 1°C 6.8g (1 R/S, 3'R)-(1'-(5-methyl-2-oxo-[1,3]-dioxol-4-ylmethoxycarbonyl)-2-oxo-[1,3']bipyrrolidinyl-3-yl)-triphenylphosphonium Bromid langsam portionsweise zudosiert. Es wurde so lange bei 1°C nachgerührt, bis das Ausgangsmaterial abreagiert hat und danach wurde das Reaktionsgemisch in 80ml Isopropanol eingerührt. Der Niederschlag wurde abfiltriert und mit Isopropanol und Methanol gewaschen. Nach dem Trocknen unter Vakuum wurde das gewünschte Produkt in Form eines Pulvers erhalten.
Auswaage: 3.30g
¹H-nmr(DMSO-*d₆*) δ 2.03 (m, 2H), 2.14 (s, 3H), 2.8-3.2(m,2H), 3.2-3.6(m,6H), 3,82(ABq, J=17,4Hz, 2H), 4.60(m, 1H), 4.80(d, 1H, J=5.0Hz), 4.90 (s, 2H), 5.00(d, 1 H, J=5.0Hz), 7.22 (s, 1 H)
MS- ESI negative mode: 519.0 (M-H, 70%)
IR (golden gate, cm⁻¹): 1815,1782,1701,1674,1411,1365

### 2. Beispiel: (6R, 7R)-7-Ammonium-3[E-(R)-1'-(5-methyl-2-oxo-[1,3]-dioxol-4-ylmethoxycarbonyl)-2-oxo-[1,3']bipyrrolidinyl-3-ylidenemethyl]-8-oxo-5-thia-1-aza-bicyclo[4.2.0]oct-2-ene-2-carbonsäure p-Toluolsulfonat

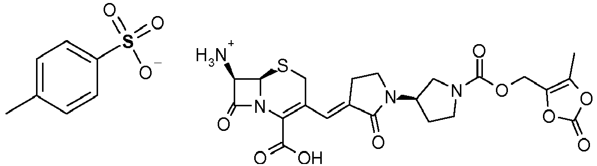

200mg (6R,7R)-7-Amino-3[E-(R)-1'-(5-methyl-2-oxo-[1,3]-dioxol-4-ylmethoxycarbonyl)-2-oxo-[1,3']bipyrrolidinyl-3-ylidenemethyl]-8-oxo-5-thia-1-aza-bicyclo[4.2.0]oct-2-ene-2-carbonsäure wurden in 3ml Methanol unter Zugabe von 95mg p-Toluolsulfonsäure Monohydrat gelöst und durch die Zugabe von 12 ml Isopropanol ausgefällt. Der Niederschlag wurde filtriert und mit Isopropanol gewaschen. AW:218mg ¹H-nmr(DMSO-*d₆*) δ 2.03 (m, 2H), 2.14 (s, 3H), 2.27 (s, 3H), 2.75-3.2(m,2H), 3.2-3.6(m,6H), 3,96(ABq, J= 17.4Hz, 2H), 4.60(m, 1 H), 4.91 (s, 2H), 5.20(d, 1 H, J=5.0Hz), 5.24(d, 1 H, J=5,0Hz), 7.10 & 7.46 (AA'BB'm,4H), 7.28 (s, 1 H) MS- ESI negative mode: 519.0 (M-H, 100%) IR (golden gate, cm⁻¹): 1785,1684, 1629,1429,1369 Das Produkt kann auch direkt aus der Reaktionsmischung durch Entsilylierung mit Isopropanol in Gegenwart von p-Toluolsulfonsäure hergestellt werden. **3. Beispiel: Dicyclohexylammonium (6R,7R)-7-Amino-3[E- (R)-1'-(5-methyl-2-oxo-[1,3]-dioxol-4-ylmethoxycarbonyl)-2-oxo-[1,3']bipyrrolidinyl-3-ylidenemethyl]-8-oxo-5-thia-1-aza-bicyclo[4.2.0]oct-2-ene-2-carboxylat**

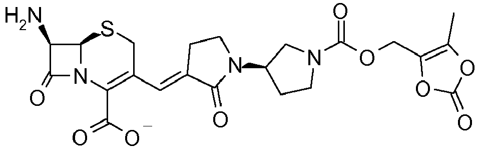

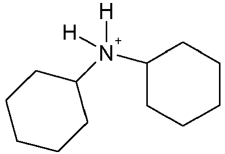

200mg (6R,7R)-7-Amino-3[E- (R)-1'-(5-methyl-2-oxo-[1,3]-dioxol-4-ylmethoxycarbonyl)-2-oxo-[1,3']bipyrrolidinyl-3-ylidenemethyl]-8-oxo-5-thia-1-aza-bicyclo[4.2.0]oct-2-ene-2-carbonsäure wurden in 3ml Methanol unter Zugabe von 95mg p-Toluolsulfonsäure Monohydrat gelöst und durch die Zugabe von 12 ml Isopropanol ausgefällt.
¹H-nmr(DMSO-*d₆*) δ 1.0-1.4(m,10H), 1.4-1.8 (m,6H), 1.8-2.1(m,6H), 2.14 (s,3H), 2.7-3.15(m,4H), 3.15-3.68m,6H), 3.68 (ABq, J= 16.8Hz, 2H), 4.59(m,2H), 4.89(m,3H), 7.33(s,1 H)
MS- ESI negative mode: 519.0 (M-H, 50%)
IR (golden gate, cm⁻¹): 2934, 2859, 1819, 1756, 1704, 1674, 1631, 1575

### 4. Referenzbeispiel:

### (6R, 7R)-7-[(Z)-2-(5-Amino-[1,2,4]thiadiazol-3-yl)-2-triphenylmethyloxyimino-acetylamino]-8-oxo-3-[(E)-(R)-2-oxo-1'-(5-methyl-2-oxo-1,3-dioxol-4ylmethoxycarbonyl)-[1,3']bipyrrolidinyl-3-ylidenemethyl]-5-thia-1-aza-bicyclo[4.2.0]oct-2-ene-2 carbonsäure

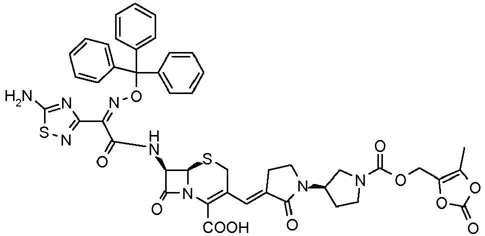

0,5 g (6R,7R)-7-Amino-3[E-(R)-1'-(5-methyl-2-oxo-1,3-dioxol-4ylmethoxycarbonyl)-2-oxo-[1,3']bipyrrolidinyl-3-ylidenemethyl]-8-oxo-5-thia-1-aza-bicyclo[4.2.0]oct-2-ene-2-carbonsäure wurden bei 0° in 20ml Dichlormethan gelöst. Anschließend wurden 0,7 ml Bis(trimethylsilyl)acetamid zugegeben sowie 0,46g 2-Trityloxyimino-2-(5-amino-1,2,4-thiadiazol-3-yl)-essigsäure chlorid Hydrochorid (J. Antibiotics 37:557 - 571, 1984) portionsweise zudosiert. Die Mischung wurde auf 15ml Wasser/15ml MeOH gegossen und die Phasen getrennt. Die Wasserphase wurde noch einmal mit 15ml Dichlormethan gewaschen und die vereinigten organischen Phasen wurden über Natriumsulfat getrocknet und im Vakuum eingeengt.
AW: 0.93g
¹H-nmr(DMSO-*d₆*) δ 1,9-2.05 (m,2H), 2.10(s,3H), 2.8-3.1(m,2H), 3.2-3.5(m,6H), 3.84(ABq, 2H, J= 17.8Hz), 4.55(m, 1H), 4.86(s, 2H), 5.22(d, 1H, J=5.1Hz), 6.02(dd, 1 H, J1= 5.1 Hz, J2=8.8Hz), 7.1-7.3(m, 16H), 8.08(br s,2H), 9.90(d,1 H, J=8.8Hz)
IR (golden gate, cm⁻¹): 1814, 1781, 1663, 1619, 1526

### 5. Beispiel: Ceftobiprol Medocaril

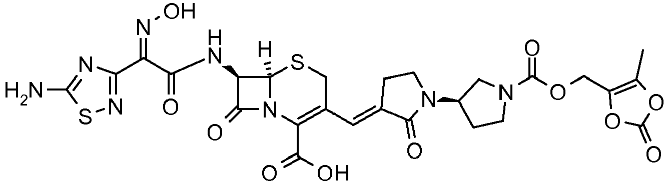

90mg (6R,7R)-7-[(Z)-2-(5-Amino-[1,2,4]thiadiazol-3-yl)-2-triphenylmethyloxyimino-acetylamino]-8-oxo-3-[(E)-(R)-2-oxo-1'-(5-methyl-2-oxo-1,3-dioxol-4ylmethoxycarbonyl)-[1,3']bipyrrolidinyl-3-ylidenemethyl]-5-thia-1-aza-bicyclo[4.2.0]oct-2-ene-2 carbonsäure wurden in 10ml Dichlormethan, 0.5ml Triethylsilan und 2ml Trifluoressigsäure gelöst und bei Raumtemperatur gerührt. Nach 30min wir die Mischung auf 150ml Diethylether getropft. Der Niederschlag wird filtriert, mit Diethylether gewaschen und im Vakuum getrocknet.
AW: 60mg
¹H-nmr(DMSO-*d₆*) δ1,9-2.1 (m,2H), 2.14(s,3H), 2.8-3.2(m,2H), 3.2-3.6(m,6H), 3.84 (ABq, 2H, J= 18.9Hz), 4.60(m, 1 H), 4.90(s, 2H), 5.16(d, 1 H, J=4.8Hz), 5.85(dd, 1 H, J1=4.8Hz, J2=8.5Hz), 7.22(s, 1H), 8.05(br s,2H), 9.47(d,1H, J=8.5Hz), 11.91(s,1H)
IR (golden gate, cm⁻¹): 1780, 1664, 1624, 1527, 1428, 1366

## Patentansprüche

1. Verfahren zur Herstellung einer Verbindung der allgemeinen Formel (1) 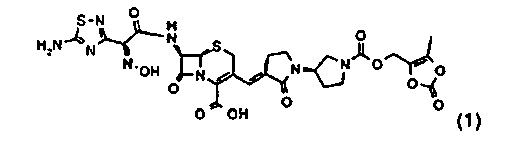 mindestens umfassend die folgenden Schritte (a) bis (c):
(a) Umsetzung einer Verbindung der allgemeinen Formel (3) 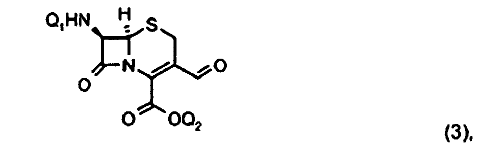 wobei Q₁ und Q₂ unabhängig voneinander für eine Silylgruppe steht oder wobei Q₁ für ein Wasserstoffatom und Q₂ für eine Silylgruppe steht,
mit einer Verbindung der allgemeinen Formel (4) 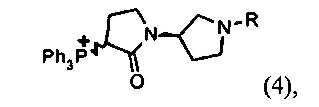 wobei R für eine 5-Methyl-2-oxo-[1,3]-dioxol-4-ylmethylcarbonyl-gruppe steht,
zu einer Verbindung der allgemeinen Formel (5) 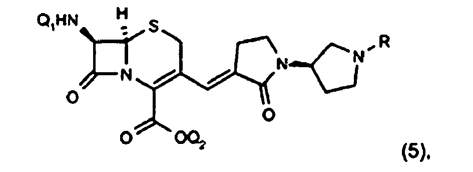 wobei Q₁, Q₂ und R wie oben definiert sind;
(b) Umsetzung der Verbindung der allgemeinen Formel (5) mit einer Verbindung der allgemeinen Formel (6) 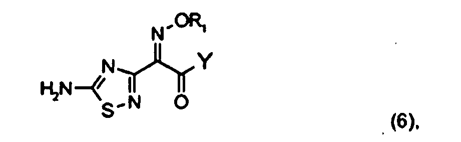 wobei R₁ für eine Hydroxy-Schutzgruppe und Y für eine aktivierende Funktionalität steht,
gegebenenfalls nach Entfernung der Schutzgruppe, falls Q₁ für eine Silylgruppe steht,
zu einer Verbindung der allgemeinen Formel (7) 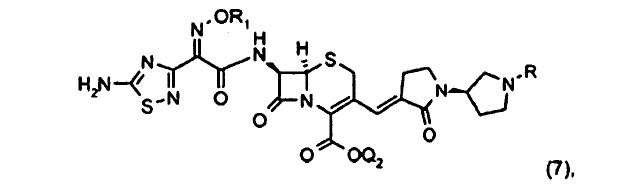 wobei R₁, Q₂ und R wie oben definiert sind, und
(c) Umsetzung der Verbindung der allgemeinen Formel (7) zu der Verbindung der allgemeinen Formel (1).

2. Verfahren nach Anspruch 1, wobei Schritt (a) in Gegenwart eines Silylierungsmittels und eines Epoxids durchgeführt wird.

3. Verfahren nach Anspruch 2, wobei es sich bei dem Silylierungsmittel um BSA handelt.

4. Verfahren nach Anspruch 3, wobei es sich bei dem Epoxid um Propylenoxid handelt.

5. Verfahren nach einem der Ansprüche 1 bis 4, wobei für die Umsetzung gemäß Schritt (b) die Verbindung der allgemeinen Formel (5) durch Silylierung oder Salzbildung gelöst wird, und nach erfolgter Acylierung mit der Verbindung der allgemeinen Formel (6) die Schutzgruppen in einem Schritt abgespalten werden.

6. Verbindung der allgemeinen Formel (5) 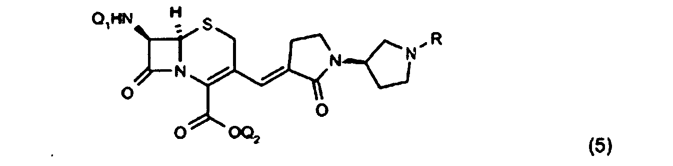 wobei Q₁ und Q₂ unabhängig voneinander für eine Silylgruppe steht oder wobei Q₁ für ein Wasserstoffatom und Q₂ für eine Silylgruppe steht und R für eine 5-Methyl-2-oxo-[1,3]-dioxol-4-ylmethylcarbonyl-gruppe steht.

7. Verbindung nach Anspruch 6, wobei Q₁ für eine Trimethylsilylgruppe und R für eine 5-Methyl-2-oxo-[1,3]-dioxol-4-ylmethylcarbonyl-gruppe steht.

8. Verbindung nach Anspruch 6 oder 7, wobei die Verbindung als Salz mit einem organischen Amin oder als Säureadditionssalz vorliegt.

9. Verwendung einer Verbindung gemäß Anspruch 6, 7 oder 8 zur Herstellung einer Verbindung der allgemeinen Formel (1).

## Claims

1. Method for the production of a compound of the general formula (1) 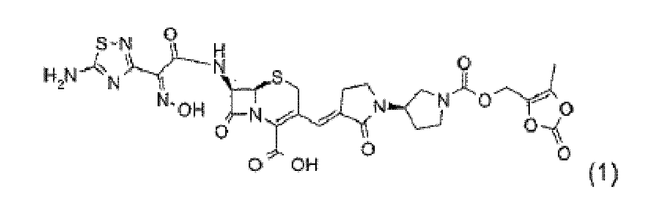 comprising at least the following steps (a) to (c) :
(a) reaction of a compound of the general formula (3) 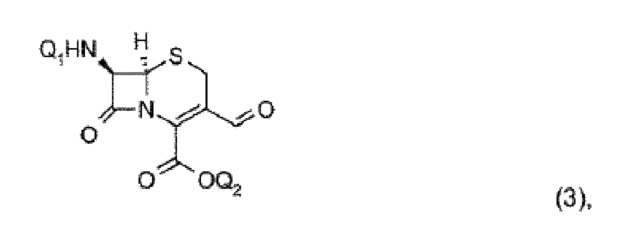 wherein Q₁ and Q₂ independently of each other represent a silyl group or wherein Q₁ represents a hydrogen atom and Q₂ represents a silyl group,
with a compound of the general formula (4) 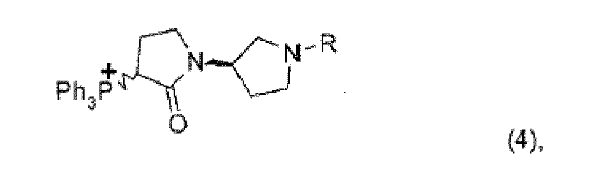 wherein R represents a 5-methyl-2-oxo-[1,3]-dioxol-4-ylmethylcarbonyl group,
to give a compound of the general formula (5) 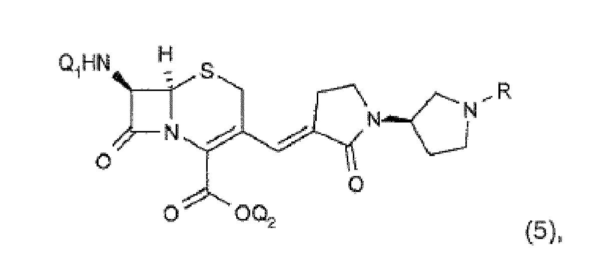 wherein Q₁, Q₂ and R are as defined above;
(b) reaction of the compound of the general formula (5) with a compound of the general formula (6) 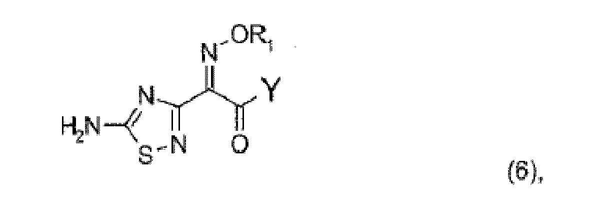 wherein R₁ represents a hydroxy-protective group and Y represents an activating functionality,
where appropriate after removal of the protective group, if Q₁ represents a silyl group,
to give a compound of the general formula (7) 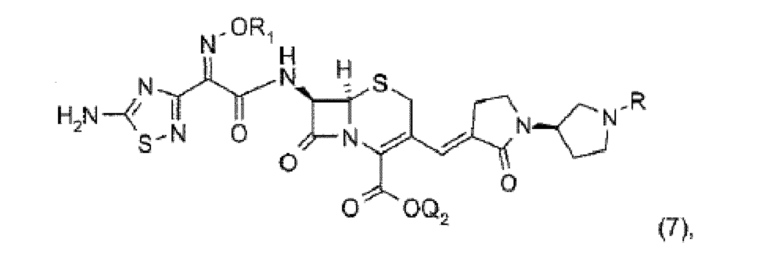 wherein R₁, Q₂ and R are as defined above, and (c) conversion of the compound of the general formula (7) into the compound of the general formula (1).

2. Method according to Claim 1, wherein step (a) is carried out in the presence of a silylating agent and an epoxide.

3. Method according to Claim 2, wherein the silylating agent is BSA.

4. Method according to Claim 3, wherein the epoxide is propylene oxide.

5. Method according to one of Claims 1 to 4, wherein for the reaction according to step (b) the compound of the general formula (5) is dissolved by silylation or salt formation, and when the acylation with the compound of the general formula (6) has been carried out, the protective groups are split off in one step.

6. Compound of the general formula (5) 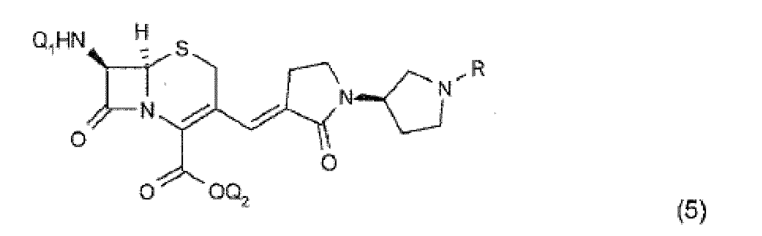 wherein Q₁ and Q₂ independently of each other represent a silyl group or wherein Q₁ represents a hydrogen atom and Q₂ represents a silyl group and R represents a 5-methyl-2-oxo-[1,3]-dioxol-4-ylmethylcarbonyl group.

7. Compound according to Claim 6, wherein Q₁ represents a trimethylsilyl group and R represents a 5-methyl-2-oxo-[1,3]-dioxol-4-ylmethylcarbonyl group.

8. Compound according to Claim 6 or 7, wherein the compound is present as a salt with an organic amine or as an acid addition salt.

9. Use of a compound according to Claim 6, 7 or 8 for the production of a compound of the general formula (1).

## Revendications

1. Procédé de fabrication d'un composé de formule générale (1) 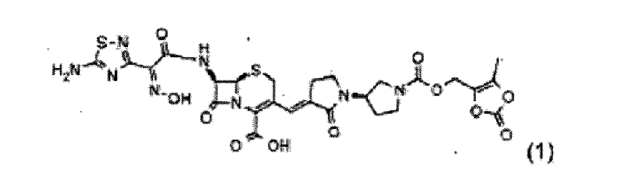 comprenant au moins les étapes (a) à (c) suivantes :
(a) la mise en réaction d'un composé de formule générale (3) 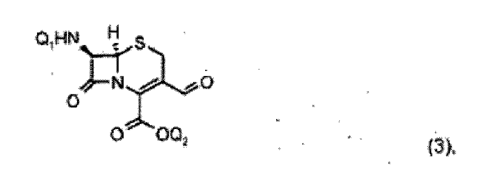 dans laquelle Q₁ et Q₂ représentent indépendamment l'un de l'autre un groupe silyle, ou
dans laquelle Q₁ représente un atome d'hydrogène et Q₂ représente un groupe silyle,
avec un composé de formule générale (4) 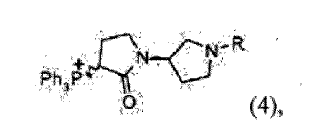 dans laquelle R représente un groupe 5-méthyle-2-oxo-[1,3]-dioxol-4-yleméthylecarbonyle,
pour former un composé de formule générale (5) 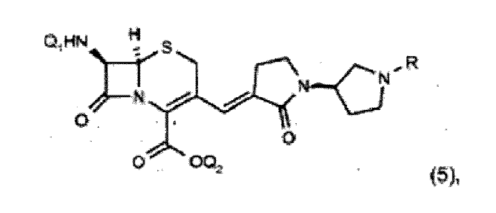 dans laquelle Q₁, Q₂ et R sont tels que définis précédemment ;
(b) la mise en réaction du composé de formule générale (5) avec un composé de formule générale (6) 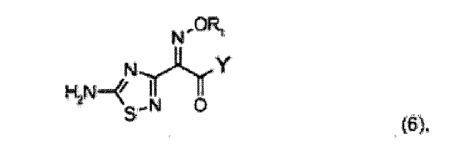 dans laquelle R₁ représente un groupe protecteur hydroxy et Y représente une fonctionnalité activante, éventuellement après élimination du groupe protecteur, si Q₁ représente un groupe silyle,
pour former un composé de formule générale (7) 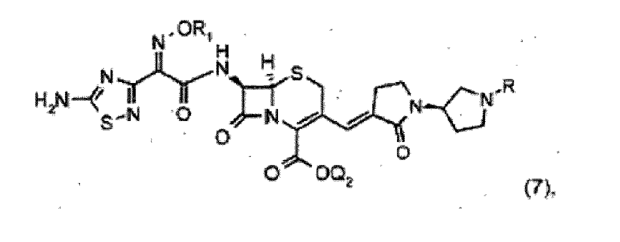 dans laquelle R₁, Q₂ et R sont tels que définis précédemment, et
(c) la mise en réaction du composé de formule générale (7) pour former le composé de formule générale (1).

2. Procédé selon la revendication 1, dans lequel l'étape (a) est réalisée en présence d'un agent de silylation et d'un époxyde.

3. Procédé selon la revendication 2, dans lequel l'agent de silylation est l'ASB.

4. Procédé selon la revendication 3, dans lequel l'époxyde est l'oxyde de propylène.

5. Procédé selon l'une quelconque des revendications 1 à 4, dans lequel, pour la réaction selon l'étape (b), le composé de formule générale (5) est dissous par silylation ou formation d'un sel, et les groupes protecteurs sont clivés en une étape après la réalisation de l'acylation avec le composé de formule générale (6).

6. Composé de formule générale (5) 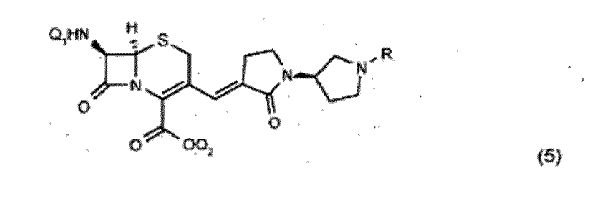 dans laquelle Q₁ et Q₂ représentent indépendamment l'un de l'autre un groupe silyle, ou dans laquelle Q₁ représente un atome d'hydrogène et Q₂ représente un groupe silyle et R représente un groupe 5-méthyle-2-oxo-[1,3]-dioxol-4-yleméthylecarbonyl.

7. Composé selon la revendication 6, dans lequel Q₁ représente un groupe triméthylsilyle et R représente un groupe 5-méthyle-2-oxo-[1,3]-dioxol-4-yleméthylecarbonyle.

8. Composé selon la revendication 6 ou 7, dans lequel le composé se trouve sous forme de sel avec une amine organique ou sous forme de sel d'addition avec un acide.

9. Utilisation d'un composé selon la revendication 6, 7 ou 8 pour la fabrication d'un composé de formule générale (1).
